(19) Europäisches Patentamt
European Patent Office
Office européen des brevets

(11) Veröffentlichungsnummer: **0 184 095**
**B1**

# (12) EUROPÄISCHE PATENTSCHRIFT

(45) Veröffentlichungstag der Patentschrift: **05.07.89**

(51) Int. Cl.⁴: **C 07 F 9/53**, C 07 F 9/65, C 08 K 5/53

(21) Anmeldenummer: **85114908.8**

(22) Anmeldetag: **25.11.85**

(54) **Bisacylphosphinoxide, ihre Herstellung und Verwendung.**

(30) Priorität: **27.11.84 DE 3443221**

(43) Veröffentlichungstag der Anmeldung: **11.06.86 Patentblatt 86/24**

(45) Bekanntmachung des Hinweises auf die Patenterteilung: **05.07.89 Patentblatt 89/27**

(84) Benannte Vertragsstaaten: **AT BE CH DE FR GB IT LI LU NL SE**

(56) Entgegenhaltungen:
**EP-A- 0 057 474**
**EP-A- 0 073 413**
**US-A- 3 668 093**

**CHEMICAL ABSTRACTS, Band 78, Nr. 9, 5. März 1973, Seite 533, Spalte 2, Zusammenfassungsnr. 58539j, Columbus, Ohio, US; P. SARTORI et al.: "Reactions of perfluoromonocarboxylic acids. VII. Trifluoroacetoxy, trifluoroacetyl and trifluoromethyl compounds of phosphorus", & Z. ANORG. ALLG. CHEM. 1972, 394(1-2), 157-170**

**Die Akte enthält technische Angaben, die nach dem Eingang der Anmeldung eingereicht wurden und die nicht in dieser Patentschrift enthalten sind.**

(73) Patentinhaber: **ESPE Stiftung & Co Produktions- und Vertriebs KG, D-8031 Seefeld (DE)**

(72) Erfinder: **Ellrich, Klaus, Dr., Bruno-Walter-Ring 34, D-8000 München 81 (DE)**
Erfinder: **Herzig, Christian, Dr., Mehringerstrasse 80, D-8263 Burghausen (DE)**

(74) Vertreter: **Abitz, Walter, Dr.-Ing. et al, Abitz, Morf, Gritschneder, Freiherr von Wittgenstein Postfach 86 01 09, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 184 095 B1

**Beschreibung**

Die Erfindung betrifft neue Bisacylphosphinoxide, ihre Herstellung sowie ihre Verwendung als Photoinitiatoren in photopolymerisierbaren Massen.

Es sind bereits eine Reihe von Photoinitiatoren auf der Basis der Acylphosphine bekannt, z.B. werden in der US-PS 3 668 093 und der DE-OS 3 020 092 Acylphosphine als Photoinitiatoren beschrieben. In den europäischen Veröffentlichungsschriften 0 073 413, 0 007 508, 0 057 474 werden Monoacylphosphinoxide als Photoinitiatoren beschrieben.

Photopolymerisierbare Massen, die mit Initiatorsystemen aus der US-PS 3 668 093 gehärtet werden, zeigen eine ungenügende Farbstabilität. Photopolymerisierbare Massen, die mit Initiatorsystemen aus der US-PS 3 668 093, der DE-OS 3 020 092 und den europäischen Veröffentlichungsschriften 0 073 423, 0 007 508 und 0 057 474 gehärtet werden, ergeben nur geringe Aushärtungstiefen und Aushärtungsgeschwindigkeiten und zeigen noch nicht befriedigende Lagerstabilitäten. Nachteilig ist ferner, dass diese Verbindungen im Bereich des sichtbaren Lichtes, d.h. Wellenlänge >400 nm, nur eine geringe Absorption haben, so dass in diesem Bereich nur geringe Lichtausbeuten erzielt werden können. Gerade die Verwendung dieses «ungefährlichen» Lichts ist aber für viele Anwendungszwecke, z.B. im dentalen Bereich, unumgänglich.

Aufgabe der Erfindung ist daher die Bereitstellung von neuen Acylphosphinoxiden.

Gegenstand der Erfindung sind Bisacylphosphinoxide der allgemeinen Formel

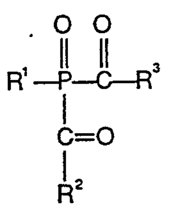

, (I)

worin bedeuten:

$R^1$ einen gradkettigen oder verzweigten $C_{1-18}$-Alkylrest,
einen Cyclohexyl-, Cyclopentyl-, Phenyl-, Naphthyl- oder Biphenylylrest,
einen Cyclopentyl-, Cyclohexyl-, Phenyl-, Naphthyl- oder Biphenylylrest, der substituiert ist durch F, Cl, Br, J, $C_{1-12}$-Alkyl und/oder $C_{1-12}$-Alkoxyl,
einen S- oder N-haltigen 5- oder 6gliedrigen heterocyclischen Ring oder
einen Rest der allgemeinen Formel:

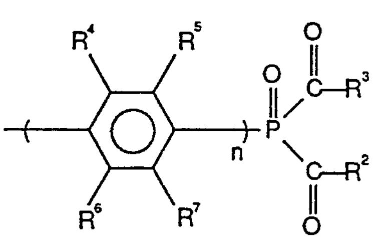

(II)

worin bedeuten:
n 1 oder 2 und
$R^4$, $R^5$, $R^6$ und $R^7$ H, $C_{1-4}$-Alkyl, $C_{1-4}$-Alkoxyl, F, Cl oder Br;
$R^2$ und $R^3$, die gleich oder verschieden sind,
einen Cyclohexyl-, Cyclopentyl-, Phenyl-, Naphthyl-, oder Biphenylylrest,
einen Cyclopentyl-, Cyclohexyl-, Phenyl-, Naphthyl-, oder Biphenylylrest, der substituiert ist durch F, Cl, Br, J, $C_{1-4}$-Alkyl und/oder $C_{1-4}$-Alkoxyl, oder
einen S- oder N-haltigen 5- oder 6gliedrigen heterocyclischen Ring; oder
$R^2$ und $R^3$ miteinander zu einem Ring verknüpft sind, der 4-10 Kohlenstoffatome enthält und durch 1-6 $C_{1-4}$-Alkylreste substituiert sein kann.

Die erfindungsgemässen Bisacylphosphinoxide zeigen eine sehr gute Reaktivität als Photoinitiatoren für photopolymerisierbare Monomere mit mindestens einer CC-Mehrfachbindung und Mischungen derselben miteinander und mit bekannten Zusatzstoffen. Die erfindungsgemässen Bisacylphosphinoxide eignen sich besonders gut als Photoinitiatoren für photopolymerisierbare Dentalmassen, wie Zahnfüllmassen, K + B-Materialien, Seal- und Bond-Lösungen. Ausserdem eignen sich die erfindungsgemässen Photoinitiatoren zur Herstellung von photopolymerisierbaren Massen, die als Formteile Verwendung finden können. Erwähnt seien weiterhin z.B. Folien, Filme oder Überzüge. Die mit den erfindungsgemässen Bisacylphosphinoxiden hergestellten photopolymerisierbaren Massen sind hinsichtlich der Farb- und Lagerstabilität und der erzielbaren Aushärtungstiefen und Aushärtungsgeschwindigkeiten den mit bisher bekannten Photoinitiatoren hergestellten photopolymerisierbaren Massen weit überlegen.

Ein weiterer Vorteil ist die geringe Sauerstoffinhibierung, bei der Photopolymerisation mit den erfindungsgemässen Bisacylphosphinoxiden.

Vorzugsweise bedeutet $R^1$ Decyl, Phenyl, Naphthyl, 4-Biphenylyl, 2-Methylphenyl, 1-Methylnaphthyl, 2,5-Dimethylphenyl 4-Propylphenyl, 4-Octylphenyl, 4-Chlorphenyl oder 4-Ethoxyphenyl.

Vorzugsweise bedeuten $R^2$ und $R^3$ Phenylreste, deren Substituenten in 2- und 6-Stellung, oder Naphthylreste, deren Substituent in 2-Stellung stehen, insbesondere bedeuten $R^2$ und $R^3$ Phenyl, Naphthyl, 2,6-Dichlorphenyl, 2,6-Dimethoxyphenyl, 2-Methylnaphthyl, 2-Methoxynaphthyl, 2,6-Dimethylphenyl oder 2,4,6-Trimethylphenyl.

Beispiele für $R^1$ = $C_{1-18}$-Alkyl sind Methyl, Propyl, i-Butyl, t-Butyl, i-Pentyl, Octyl und Decyl.

Beispiele für $C_{1-12}$-Alkylreste in $R_1$ = alkyl- oder alkoxylsubstituierter Cyclopentyl-, Cyclohexyl-, Phenyl-, Naphthyl- oder Biphenylylrest sind Methyl, Ethyl, n-Propyl, i-, t- oder n-Butyl, Pentyl, Octyl und Decyl.

Beispiele für $C_{1-4}$-Alkylreste sind Methyl, Ethyl, Propyl, i-Propyl, i-Butyl, t-Butyl und n-Butyl.

Als Beispiele für erfindungsgemässe Bisacylphosphinoxide seien genannt:
Bis(2,6-dichlorbenzoyl)-phenylphosphinoxid

Bis(2,6-dichlorbenzoyl)-2,5-dimethylphenylphosphinoxid
Bis(2,6-dichlorbenzoyl)-4-ethoxyphenylphosphinoxid
Bis(2,6-dichlorbenzoyl)-4-biphenylylphosphinoxid
Bis(2,6-dichlorbenzoyl)-4-n-propylphenylphosphinoxid
Bis(2,6-dichlorbenzoyl)-2-naphthylphosphinoxid
Bis(2,6-dichlorbenzoyl)-1-naphthylphosphinoxid
Bis(2,6-dichlorbenzoyl)-4-chlorphenylphosphinoxid
Bis(2,6-dichlorbenzoyl)-2,4-dimethoxyphenylphosphinoxid
Bis(2,6-dichlorbenzoyl)-decylphosphinoxid
Bis(2,6-dichlorbenzoyl)-4-octylphenylphosphinoxid
Bis(2,6-dimethoxybenzoyl)-2,5-dimethylphenylphosphinoxid
Bis(2,6-dimethoxybenzoyl)-phenylphosphinoxid
Bis(2,4,6-trimethylbenzoyl)-2,5-dimethylphenylphosphinoxid
Bis(2,6-dichlor-3,4,5-trimethoxybenzoyl)-2,5-dimethyl-phenylphosphinoxid
Bis(2,6-dichlor-3,4,5-trimethoxybenzoyl)-4-ethoxyphenylphosphinoxid
Bis(2-methyl-1-naphthoyl)-2,5-dimethylphenylphosphinoxid
Bis(2-methyl-1-naphthoyl)-phenylphosphinoxid
Bis(2-methyl-1-naphthoyl)-4-biphenylylphosphinoxid
Bis(2-methyl-1-naphthoyl)-4-ethoxyphenylphosphinoxid
Bis(2-methyl-1-naphthoyl)-2-naphthylphosphinoxid
Bis(2-methyl-1-naphthoyl)-4-propylphenylphosphinoxid
Bis(2-methoxy-1-naphthoyl)-2,5-dimethylphosphinoxid
Bis(2-methoxy-1-naphthoyl)-4-ethoxyphenylphosphinoxid
Bis(2-methoxy-1-naphthoyl)-4-biphenylylphosphinoxid
Bis(2-methoxy-1-naphthoyl)-2-naphthylphosphinoxid
Bis(2-chlor-1-naphthoyl)-2,5-dimethylphenylphosphinoxid

Die Herstellung der Bisacylphosphinoxide mit Formel I erfolgt erfindungsgemäss durch Oxidation der zum Teil bereits bekannten Bisacylphosphine. Als Oxidationsmittel dienen z.B. $O_2$, $NO_2$, $H_2O_2$ und andere dem Fachmann geläufige Oxidationsmittel.

Die gewünschten Bisacylphosphine erhält man beispielsweise durch Reaktion der monosubstituierten Phosphine der Formel $R^1PH_2$ mit stöchiometrischen Mengen Acylchlorid $R^2COCl$, $R^3COCl$. ($R^1$, $R^2$ und $R^3$ haben die im Patentanspruch 1 beschriebenen Bedeutungen). Hierbei erfolgt die HCl-Eliminierung in an sich bekannter Weise, vorteilhaft unter Verwendung einer Hilfsbase, z.B. eines teritären Amins.

Als photopolymerisierbare Monomere eignen sich die dem Fachmann bekannten Verbindungen und Stoffe mit polymerisierbaren CC-Doppelbindungen, die vorteilhafterweise durch z.B. Aryl-, Carbonyl-, Ester-, Carboxy- oder Cyanidgruppen aktiviert sind. Genannt seien beispielsweise Acrylsäure und Methacrylsäure sowie deren Ester mit ein- oder mehrwertigen Alkoholen mit bis zu 20 Kohlenstoffatomen, wie Methylmethacrylat, Ethylmethacrylat, Triethylenglykoldimethacrylat. Weiterhin verwendet werden können Acryl- und Methacrylderivate des Bisphenol A, z.B. die in der DE-PS 1 921 869 und der US-PS 3 066 112 genannten Monomere.

Verwendet werden können ausserdem Alkandioldiacrylate und Alkandioldimethacrylate, wie 1,6-Hexandioldi(meth)-acrylat, 1,4-Butandioldi(meth)acrylat, Tri- oder Tetraethylenglykoldi(meth)acrylat und die in der DE-PS 2 816 823 genannten Diacrylsäure- und Dimethacrylsäureester des Bis-hydroxymethyltricyclo[5.2.1.0.2,6]-decans. Weiterhin verwendet werden können die Reaktionsprodukte aus Diisocyanaten und Hydroxyalkyl(meth)acrylaten, wie sie beispielsweise in der DE-OS 2 312 559 beschrieben sind, Addukte aus Diisocyanaten und 2,2-Propan-bis-3-(4-phenoxy)-1,2-hydroxypropan-1-methacrylat nach der US-PS 3 629 187 sowie die Addukte aus Isocyanaten und Methacroylalkylethern, -alkoxybenzolen bzw. -alkoxycycloalkanen, wie sie in der europäischen Veröffentlichungsschrift 44 352 beschrieben sind, verwendet werden.

Selbstverständlich können auch Gemische aus geeigneten Monomeren verwendet werden.

Weiterhin können aromatische Vinylverbindungen, wie Styrol und dessen Derivate, z.B. α-Alkylderivate des Styrols, wie α-Methylstyrol und Vinyltoluol, verwendet werden.

Als polymerisierbare höhermolekulare Verbindungen sind beispielsweise geeignet: Ungesättigte Polyester, hergestellt durch Umsetzung von α, β ungesättigten Dicarbonsäuren – gegebenenfalls im Gemisch mit gesättigten Dicarbonsäuren – mit Alkandiolen.

Den photopolymerisierbaren Verbindungen, deren Zusammensetzung für den jeweiligen Verwendungszweck dem Fachmann geläufig ist, können in bekannter Weise gesättigte und/oder ungesättigte Polymere sowie weitere Zusatzstoffe, wie Inhibitoren gegen die thermische Polymerisation, Pigmente, Farbstoffe, Peroxide und Füllstoffe, zugesetzt sein. Solche Gemische sind dem Fachmann bekannt. Art und Menge der Zusätze hängen vom jeweiligen Verwendungszweck ab. Die erfindungsgemässen Bisacylphosphinoxide werden dabei im allgemeinen in einer Konzentration von 0,01-15 Gew.-%, vorzugsweise 0,05-5 Gew.-%, bezogen auf die photopolymerisierbare Masse, eingesetzt. Sie können gegebenenfalls mit Beschleunigern und/oder anderen Photoinitiatoren kombiniert werden. Dem Fachmann bekannte Beschleuniger sind z.B. sekundäre und/oder tertiäre Amine, Phosphite, Sulfin- und Barbitursäurederivate. Ferner können die Bisacylphosphinoxide, gegebenenfalls in Anwesenheit der oben bezeichneten Beschleuniger, in Kombination mit anderen Photoinitiatoren zur

Lichthärtung photopolymerisierbarer Massen eingesetzt werden. Solche anderen Photoinitiatoren sind z. B. aromatische Ketone, wie Benzilketale, Benzoinether, Benzoinester, Thioxanthone und 1,2-Diketone, z. B. Campherchinon.

Als Strahlungsquellen für das die Polymerisation solcher Mischungen auslösende Licht verwendet man im allgemeinen solche, die Licht vorzugsweise im Absorptionsbereich der erfindungsgemässen Verbindung aussenden, d.h. zwischen 200 und 500 nm. Besonders geeignet für die Aushärtung von Dentalmassen ist Licht einer Wellenlänge zwischen 400 und 500 nm. Besonders geeignet sind hierfür gegebenenfalls dotierte Quecksilber-Niederdruck-, Mitteldruck- und Hochdruckstrahler, superaktinische Leuchtstoffröhren, Impulsstrahler sowie Glühlampen, beispielsweise Halogenlampen.

Ein besonderer Vorteil der erfindungsgemässen Bisacylphosphinoxide ist, dass sie sich als Photoinitiatoren eignen, mit denen die Photopolymerisation mit längerwelligeren und damit ungefährlicheren Lichtquellen, wie Leuchtstoffröhren, oder die Härtung mit Sonnenlicht möglich ist.

Beispiel 1 (Herstellung)

a) Bis(2,6-dichlorbenzoyl)-2,5-dimethylphenylphosphin

Zu 46,6 g 2,6-Dichlorbenzoylchlorid in 60 ml Toluol gibt man bei 20 °C 13,8 g 2,5-Dimethylphenylphosphin. Das Gemisch wird auf 90 °C erhitzt, worauf man innerhalb von 5 Minuten 22,2 g trockenes Triethylamin zugibt. Zur Vervollständigung der Umsetzung rührt man bei gleicher Temperatur noch 5 Stunden. Nach Verdünnung des Reaktionsgemisches wäscht man 2× mit Wasser und verdünnter Bicarbonatlösung, trocknet die organische Phase und engt sie im Vakuum ein. Man erhält die Titelverbindung als gelbe kristalline Masse mit einer Reinheit von > 95% (HPLC).
Ausbeute: 50 g

b) Bis(2,6-dichlorbenzoyl)-2,5-dimethylphenylphosphinoxid

Das rohe Bis(2,6-dichlorbenzoyl)-2,5-dimethylphenylphosphin (50 g) wird in 1 l Acetonitril gelöst, mit 150 ml 30%igem Wasserstoffperoxid versetzt und 1 Stunde auf 60 °C erwärmt. Unter Farbvertiefung bildet sich das Phosphinoxid, welches nach Wasserzugabe und Abkühlen kristallin ausfällt. Umkristallisation aus Acetonitril/Wasser liefert 37 g der Titelverbindung (74% d.Th. über 2 Stufen)

Fp: 172 °C
UV: $\lambda$ max. = 366 nm ($\varepsilon_{mol}$ = 1065)
31 $_{P-NMR}$: $\delta$ = + 9,1 ppm (gegen $H_3PO_4$ ext.)
1 $_{H-NMR}$: $\delta$ = 1,30 ppm (s; 3 H)
2,65 ppm (d, J(PCCCH) = 1 Hz; 3H)
7,05–7,35 ppm (m; 8 H)
7,26 ppm (d, J(PCCH) = 13 Hz; 1 H)
IR: $\gamma$ (C = O) = 1703 $cm^{-1}$
$\gamma$ (P = O) = 1200 $cm^{-1}$
Elementaranalyse:
$C_{22}H_{15}Cl_4O_3P$ (500,14)
berechnet:
C 52,83% H 3,02% Cl 28,36%
gefunden:
C 52,72% H 3,07% Cl 28,04%

Analog werden die in Tabelle 1 aufgeführten Verbindungen erhalten.

Tabelle 1

| | Schmelzpunkt | $^{31}$P-NMR | UV $\lambda_{max.}$ | ($\varepsilon_{mol}$) | $\varepsilon_{mol}$ bei 400 nm |
|---|---|---|---|---|---|
| Bis(2,6-dichlorbenzoyl)-phenylphosphinoxid | 193–194 °C | 2,9 ppm | 363 nm | (1130) | 607 |
| Bis(2,6-dichlorbenzoyl)-2,5-dimethylphenyl-phosphinoxid | 161–164 °C | 9,1 ppm | 360 nm | (1065) | 580 |
| Bis(2,6-dichlorbenzoyl)-4-ethoxyphenyl-phosphinoxid | 173–174 °C | 4,2 ppm | 364 nm | (1640) | 690 |
| Bis(2,6-dichlorbenzoyl)-4-octylphenylphosphinoxid | 141–142 °C | 4,1 ppm | 390 nm | (670) | 620 |
| Bis(2,6-dichlorbenzoyl)-decylphosphinoxid | 95–96 °C | 25,9 ppm | 407 nm | (480) | 470 |
| Bis(2,6-dichlorbenzoyl)-4-biphenyl phosphinoxid | 209–210 °C | 3,2 ppm | 364 nm | (2060) | 800 |
| Bis(2,6-dichlorbenzoyl)-2,5-dimethyl-phenylphosphinoxid | 50 °C* | 17,6 ppm | – | | 505 |
| Bis(2,6-dichlorbenzoyl)-phenylphosphinoxid | 174–176 °C | 8,6 ppm | – | | 550 |
| Bis(2,6-dichlor-3,4,5-trimethoxybenzoyl)-2,5-dimethylphenylphosphinoxid | 123–124 °C | 8,7 ppm | – | | 640 |
| Bis(2-methyl-1-naphthoyl)-phenylphosphinoxid | | 8,8 ppm | 323 nm | (4900) | 1040 |
| Bis(2-methyl-1-naphthoyl)-4-biphenyl-phosphinoxid | 189–193 °C | 9,1 ppm | 350 nm | (5600) | 1680 |
| Bis(2-methoxy-1-naphthoyl)2,5-dimethylphenyl-phosphinoxid | 197–199 °C | 21,0 ppm | – | | 2550 |
| Bis(2-methoxy-1-naphthoyl)-4-ethoxyphenyl-phosphinoxid | 191–192 °C | 12,2 ppm | – | | 2700 |

* Erweichungspunkt

Beispiel 2 (Verwendung)

70 Gewichtsteile Bis-acryloxymethyl-tricyclo-[5.2.1.0.2,6]-decan und
30 Gewichtsteile 2,2-Bis-4-(3-methacryloxy-2-hydroxypropoxy)phenylpropan (Bis-GMA)
werden unter vorsichtigem Erwärmen so lange gerührt, bis eine klare Lösung I entsteht.

Zu der auf Raumtemperatur abgekühlten Lösung I werden 0,5 Gewichtsprozent Photoinitiator

gegeben und so lange gerührt, bis eine klare Lösung vorliegt.

Die erhaltenen Lösungen werden in einen zylindrischen Körper (∅ 5mm, Länge 8 mm und bei grösseren Schichtdicken 20 mm) eingefüllt. Anschliessend belichtet man mit einem handelsüblichen dentalen Bestrahlungsgerät (Elipar-Visio/Espe) 20 Sekunden lang, nimmt das Polymerisat aus dem Zylinder, entfernt die weichen oder gelartigen, nicht durchpolymerisierten Bestandteile mit einem Kunststoffspatel und misst die erzielte Schichtdicke. Hierzu werden die Lösungen vor der Polymerisation 1 Tag bzw. 1 Monat unter Lichtausschluss gelagert. Die Ergebnisse sind in Tabelle 2 aufgelistet.

Tabelle 2

| Photoinitiator | Schichtdicke (mm) nach 24 h | 1 Monat | Prozentualer Schichtdickenabfall | Extinktion bei 400 nm |
|---|---|---|---|---|
| 2,6-Dichlorbenzoyl-diphenylphosphinoxid (eur. Veröffentlichungsschrift 7 508) | 7 | 6,5 | 7% | 190 |
| 2,6-Dichlorbenzoyl-bis(2,5-dimethylphenyl)-phosphinoxid | 5,1 | 4,1 | 20% | 440 |
| Bis(2,6-dichlorbenzoyl)-phenyl phosphinoxid (erfindungsgemäss) | 15 | 14,5 | 3% | 607 |
| Bis(2,6-dichlorbenzoyl)-2,5-dimethylphenylphosphinoxid (erfindungsgemäss) | 15 | 14,5 | 3% | 580 |

Die erfindungsgemässen Bisacylphosphinoxide weisen gegenüber den im Stand der Technik bekannten Monoacylphosphinoxiden überraschend erhöhte Extinktion im Wellenbereich >400 nm auf, erzielen mehr als die doppelte Schichtdicke und sind auch nach einem Monat in ihrer Leistung lediglich um 3% (gegenüber 7–20% bei den Monoacylphosphinoxiden) abgefallen.

Beispiel 3 (Verwendung)

Herstellung einer photopolymerisierbaren Zahnfüllmasse (Composite).

Aus 35 Gewichtsteilen Bis-acryloxymethyl-tricyclo-[5.2.1.0.2,6]-decan

15 Gewichtsteilen Bis-GMA und

7 Gewichtsteilen silanisierter pyrogener Kieselsäure wird eine Vormischung geknetet.

1,84 g dieser Vormischung und 1,66 g Lösung I aus Beispiel 2 werden 5 Minuten vorgeknetet. Anschliessend werden in kleinen Portionen insgesamt 15 g silanisierter und zahnähnlich pigmentierter Quarz (mittlere Korngrösse ca. 6 µm) zugegeben und zu einer Zahnfüllmasse mit einheitlicher pastöser Konsistenz verknetet. Füllt man die Paste in die im Beispiel 2 beschriebene Form, so erhält man nach 20 sec. Belichtung eine durchpolymerisierte Schichtdicke von 5 mm. Die Druckfestigkeit des Polymerisats beträgt 300 MPa.

Beispiel 4 (Verwendung)

Zahnfüllmassen, die nach Beispiel 3 unter Verwendung der in Tabelle 3 angegebenen Photoinitiatoren hergestellt wurden, werden in zylindrische Körper (∅ 3 mm, Höhe 3 mm) gefüllt, in deren Mitte mit einem Temperaturfühler die Temperatur während der Polymerisation gemessen werden kann. Nach 20 Sekunden Belichtungszeit mit einem handelsüblichen dentalen Bestrahlungsgerät (Elipar-Visio-Lampe/Espe) werden die Körper entnommen und mit Toluol die Schmierschicht entfernt. Aus der Gewichtsdifferenz wird die Schmierschichtmenge in mg/cm² jeweils an der oberen und unteren Zylinderfläche errechnet. Ausserdem wird der zeitliche Temperaturverlauf mit dem Thermofühler und einem angeschlossenen Messinstrument verfolgt. Ergebnisse sind nachfolgend angegeben.

Tabelle 3

| Photoinitiator | $T_{max.}$ (°C) | Zeit zur Erreichung der $T_{max.}$ (sec.) | Schmierschicht (mg/cm²) |
|---|---|---|---|
| 2,4,6-Trimethylbenzoyl-diphenylphosphinoxid (eur. Veröffentlichungsschrift 7 508) | 39,8 | 18 | 1,8/2,0 |
| Bis(2,6-dichlorbenzoyl)-2,5-dimethylphenylphosphinoxid (erfindungsgemäss) | 43,7 | 16 | 1,0/1,1 |

Es zeigt sich, dass das erfindungsgemässe Bisacylphosphinoxid gegenüber dem Initiator des Stands ser Technik eine höhere Polymerisationsgeschwindigkeit und geringe Sauerstoffinhibierung aufweist.

**Patentansprüche für die Vertragsstaaten BE, CH, DE, FR, GB, IT, LI, LU, NL, SE**

1. Bisacylphosphinoxide der allgemeinen Formel

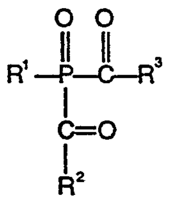

(I)

worin bedeuten:
$R^1$ einen gradkettigen oder verzweigten $C_{1-18}$-Alkylrest,
einen Cyclohexyl-, Cyclopentyl-, Phenyl-, Naphthyl- oder Biphenylylrest,
einen Cyclopentyl-, Cyclohexyl-, Phenyl-, Naphthyl- oder Biphenylylrest, der substituiert ist durch F, Cl, Br, J, $C_{1-12}$-Alkyl und/oder $C_{1-12}$-Alkoxyl,
einen S- oder N-haltigen 5- oder 6gliedrigen heterocyclischen Ring oder einen Rest der allgemeinen Formel:

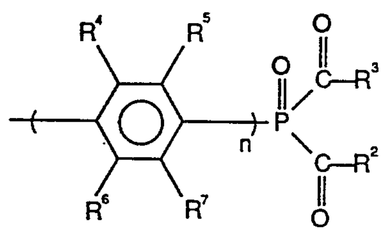

(II)

worin bedeuten:
n 1 oder 2 und
$R^4$, $R^5$, $R^6$ und $R^7$ H, $C_{1-4}$-Alkyl, $C_{1-4}$-Alkoxyl, F, Cl oder Br;
$R^2$ und $R^3$, die gleich oder verschieden sind,
einen Cyclohexyl-, Cyclopentyl-, Phenyl-, Naphthyl- oder Biphenylylrest,
einen Cyclopentyl-, Cyclohexyl-, Phenyl-, Naphthyl- oder Biphenylylrest, der substituiert ist durch F, Cl, Br, J, $C_{1-4}$-Alkyl und/oder $C_{1-4}$-Alkoxyl, oder
einen S- oder N-haltigen 5- oder 6gliedrigen heterocyclischen Ring; oder
$R^2$ und $R^3$ miteinander zu einem Ring verknüpft sind, der
4–10 Kohlenstoffatome enthält und durch
1–6 $C_{1-4}$-Alkylreste substituiert sein kann.

2. Verbindungen gemäss Anspruch 1, dadurch gekennzeichnet, dass $R^1$ Decyl, Phenyl, Naphthyl, 4-Biphenylyl, 2-Methylphenyl, 1-Methylnaphthyl, 2,5-Dimethylphenyl, 4-Propylphenyl, 4-Octylphenyl, 4-Chlorphenyl oder 4-Ethoxyphenyl bedeutet.

3. Verbindungen gemäss Anspruch 1 oder 2, dadurch gekennzeichnet, dass $R^2$ und $R^3$ Phenylreste, deren Substituenten in 2- und 6-Stellung, oder Naphthylreste, deren Substituent in 2-Stellung stehen, bedeuten.

4. Verbindungen gemäss Anspruch 1 oder 2, dadurch gekennzeichnet, dass $R^2$ und $R^3$ Phenyl, Naphthyl, 2,6-Dichlorphenyl, 2,6-Dimethoxyphenyl, 2-Methylnaphthyl, 2-Methoxynaphthyl, 2,6-Dimethylphenyl oder 2,4,6-Trimethylphenyl bedeuten.

5. Verbindung gemäss einem der Ansprüche 1 bis 4, dadurch gekennzeichnet, dass $R^2$ und $R^3$ die gleiche Bedeutung aufweisen.

6. Bis(2,6-dichlorbenzoyl)-phenylphosphinoxid.

7. Bis(2,6-dichlorbenzoyl)-2,5-dimethylphenylphosphinoxid.

8. Bis(2,6-dichlorbenzoyl)-4-n-propylphenylphosphinoxid.

9. Verfahren zur Herstellung der Verbindungen des Anspruchs 1, dadurch gekennzeichnet, dass man ein Bisacylphosphin der allgemeinen Formel

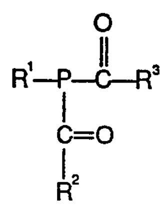

(III)

oxidiert.

10. Verwendung der Verbindungen des Anspruchs 1 als Photoinitiatoren in photopolymerisierbaren Massen.

**Patentansprüche für den Vertragsstaat AT**

1. Verfahren zur Herstellung von Bisacylphosphinoxiden der allgemeinen Formel

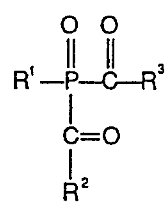

(I)

worin bedeuten:
$R^1$ einen gradkettigen oder verzweigten $C_{1-18}$-Alkylrest, einen Cyclohexyl-, Cyclopentyl-, Phenyl-, Naphthyl- oder Biphenylylrest,
einen Cyclopentyl-, Cyclohexyl-, Phenyl-, Naphthyl- oder Biphenylylrest, der substituiert ist durch F, Cl, Br, J, $C_{1-12}$-Alkyl und/oder $C_{1-12}$-Alkoxyl,
einen S- oder N-haltigen 5- oder 6gliedrigen heterocyclischen Ring oder einen Rest der allgemeinen Formel:

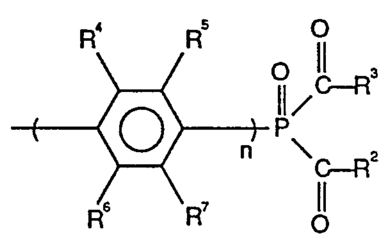

(II)

worin bedeuten:
n 1 oder 2 und
$R^4$, $R^5$, $R^6$ und $R^7$ H, $C_{1-4}$-Alkyl, $C_{1-4}$-Alkoxyl, F, Cl oder Br;

$R^2$ und $R^3$, die gleich oder verschieden sind, einen Cyclohexyl-, Cyclopentyl-, Phenyl-, Naphthyl- oder Biphenylylrest, einen Cyclopentyl-, Cyclohexyl-, Phenyl-, Naphthyl- oder Biphenylylrest, der substituiert ist durch F, Cl, Br, J, $C_{1-4}$-Alkyl und/oder $C_{1-4}$-Alkoxyl, oder einen S- oder N-haltigen 5- oder 6gliedrigen heterocyclischen Ring; oder $R^2$ und $R^3$ miteinander zu einem Ring verknüpft sind, der 4-10 Kohlenstoffatome enthält und durch 1-6 $C_{1-4}$-Alkylreste substituiert sein kann, dadurch gekennzeichnet, dass man ein Bisacylphosphin der allgemeinen Formel

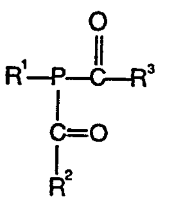

(III)

worin $R^1$, $R^2$ und $R^3$ die oben angegebenen Bedeutungen haben, oxidiert.

2. Verfahren gemäss Anspruch 1, dadurch gekennzeichnet, dass man Verbindungen III umsetzt, in denen $R^1$ Decyl, Phenyl, Naphthyl, 4-Biphenylyl, 2-Methylphenyl, 1-Methylnaphthyl, 2,5-Dimethylphenyl, 4-Propylphenyl, 4-Octylphenyl, 4-Chlorphenyl oder 4-Ethoxyphenyl bedeutet.

3. Verfahren gemäss Anspruch 1 oder 2, dadurch gekennzeichnet, dass man Verbindungen III umsetzt, in denen $R^2$ und $R^3$ Phenylreste, deren Substituenten in 2- und 6-Stellung, oder Naphthylreste, deren Substituent in 2-Stellung stehen, bedeuten.

4. Verfahren gemäss Anspruch 1 oder 2, dadurch gekennzeichnet, dass man Verbindungen III umsetzt, in denen $R^2$ und $R^3$ Phenyl, Naphthyl, 2,6-Dichlorphenyl, 2,6-Dimethoxyphenyl, 2-Methylnaphthyl, 2-Methoxynaphthyl, 2,6-Dimethylphenyl oder 2,4,6-Trimethylphenyl bedeuten.

5. Verfahren gemäss einem der Ansprüche 1 bis 4, dadurch gekennzeichnet, dass man Verbindungen III umsetzt, in denen $R^2$ und $R^3$ die gleiche Bedeutung aufweisen.

6. Verfahren nach Anspruch 1, dadurch gekennzeichnet, dass man Bis(2,6-dichlorbenzoyl)-phenylphosphinoxid herstellt.

7. Verfahren nach Anspruch 1, dadurch gekennzeichnet, dass man Bis(2,6-dichlorbenzoyl)-2,5-dimethylphenylphosphinoxid herstellt.

8. Verfahren nach Anspruch 1, dadurch gekennzeichnet, dass man Bis(2,6-dichlorbenzoyl)-4-n-propylphenylphosphinoxid herstellt.

9. Verwendung der gemäss Anspruch 1 hergestellten Verbindungen als Photoinitiatoren in photopolymerisierbaren Massen.

**Revendications pour les Etats contractants BE, CH, DE, FR, GB, IT, LI, LU, NL, SE**

1. Oxyde de bis-acyl phosphine de formule générale

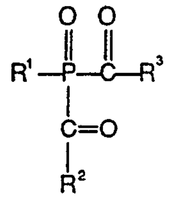

(I)

dans laquelle:
$R^1$ est un reste alkyle en $C_1$ à $C_{18}$, à chaîne linéaire ou ramifiée; un reste cyclohexyle, un reste cyclopentyle; un reste phényle, un reste naphtyle ou un reste biphénylyle; un reste cyclopentyle, cyclohexyle, phényle, naphtyle ou biphénylyle, qui est substitué par F, Cl, Br, I, alkyle en $C_1$ à $C_{12}$ et/ou alkoxy en $C_1$ à $C_{12}$; un noyau hétérocycle à 5 ou 6 atomes renfermant S ou N, ou bien un reste de formule générale:

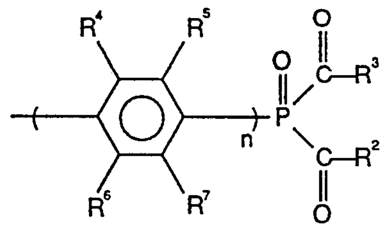

(II)

n = 1 ou 2, et $R^4$, $R^5$, $R^6$ et $R^7$ sont H, alkyle en $C_1$ à $C_4$, alkoxy en $C_1$ à $C_4$, F, Cl ou Br;
$R^2$ et $R^3$, qui sont semblables ou différents sont: un reste cyclohexyle, cyclopentyle, phényle, naphtyle ou biphénylyle; un reste cyclopentyle, cyclohexyle, phényle, naphtyle ou biphénylyle, qui est substitué par F, Cl, I, Br, alkyle en $C_1$ à $C_4$ et/ou alkoxy en $C_1$ à $C_4$; ou un noyau hétérocyclique à 5 ou 6 atomes renfermant S ou N; ou bien $R^2$ et $R^3$ sont reliés ensemble pour former un noyau, qui renferme de 4 à 10 atomes de carbone et peut être substitué par 1 à 6 restes alkyle en $C_1$ à $C_4$.

2. Composés selon la revendication 1, caractérisés en ce que $R^1$ est décyle, phényle, naphtyle, biphénylyle-4, méthyl-2 phényle, méthyl-1 naphtyle, diméthyl-2,5 phényle, propyl-4 phényle, octyl-4 phényle, chloro-4 phényle, ou éthoxy-4 phényle.

3. Composés selon la revendication 1 ou 2, caractérisés en ce que $R^2$ et $R^3$ sont des restes phényle dont les substituants se trouvent dans les positions 2 et 6, ou des restes naphtyle dont le substituant se situe en position 2.

4. Composés selon la revendication 1 ou 2, caractérisés en ce que $R^2$ et $R^3$ sont phényle, naphtyle, dichloro-2,6 phényle, diméthoxy-2,6 phényle, méthyl-2 naphtyle, méthoxy-2 naphtyle, diméthyl-2,6 phényle ou triméthyl-2,4,6 phényle.

5. Composés selon une des revendications 1 à 4, caractérisés en ce que $R^2$ et $R^3$ présentent la même signification.

6. Oxyde de phényl bis(dichloro-2,6 benzoyl) phosphine.

7. Oxyde de diméthyl-2,5 phényl bis-(dichloro-2,6 benzoyl) phosphine.

8. Oxyde de n-propyl-4 phényl bis-(dichloro-2,6 benzoyl) phospine.

9. Procédé de préparation des composés selon la revendication 1, caractérisé en ce que l'on oxyde une bis-acyl phosphine de formule générale

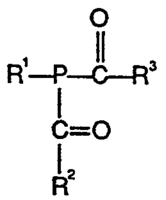

(III)

10. Utilisation des composés selon la revendication 1 en tant que photo-initiateurs dans des masses photopolymérisables.

**Revendications pour Etat contractant AT**

1. Procédé de préparation d'oxydes de bis-acyl phosphines de formule générale

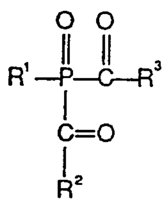

(I)

dans laquelle
$R^1$ est un reste alkyle en $C_1$ à $C_{18}$ à chaîne linéaire ou ramifiée; un reste cyclohexyle, cyclopentyle, phényle, naphtyle ou biphénylyle; un reste cyclopentyle, cyclohexyle, phényle, naphtyle ou biphénylyle, qui est substitué par F, Cl, Br, I, alkyle en $C_1$ à $C_{12}$ et/ou alkoxy en $C_1$ à $C_{12}$; un noyau hétérocyclique à 5 ou 6 atomes de carbone renfermant S ou N; ou bien un reste de formule générale:

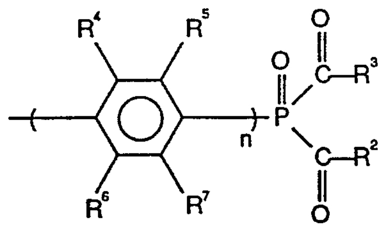

(II)

dans laquelle n = 1 ou 2, et $R^4$, $R^5$, $R^6$ et $R^7$ sont H, alkyle en $C_1$ à $C_4$, alkoxy en $C_1$ à $C_4$, F, Cl ou Br;
$R^2$ et $R^3$, qui sont semblables ou différents, sont: un reste cyclohexyle, cyclopentyle, phényle, naphtyle ou bipénylyle; un reste cyclopentyle, cyclohexyle, phényle, naphtyle ou biphénylyle, qui est substitué par F, Cl, I, Br, alkyle en $C_1$ à $C_4$ et/ou alkoxy en $C_1$ à $C_4$; ou un noyau hétérocyclique à 5 ou 6 atomes de carbone renfermant S ou N; ou bien, $R^2$ et $R^3$ sont reliés ensemble pour former un noyau qui renferme de 4 à 10 atomes de carbone et peut être substitué par 1 à 6 restes alkyle en $C_1$ à $C_4$, caractérisé en ce qu'on oxyde une bis-acyl phosphine de formule générale

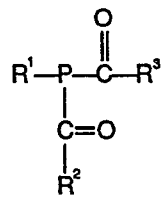

(III)

dans laquelle $R^1$, $R^2$ et $R^3$ ont les significations indiquées plus haut.

2. Procédé selon la revendication 1, caractérisé en ce qu'on fait réagir des composés III dans lesquels $R^1$ est décyle, phényle, naphtyle, biphénylyl-4, méthyl-2 phényle, méthyl-1 naphtyle, diméthyl-2,5 phényle, propyl-4 phényle, octyl-4 phényle, chloro-4 phényle ou éthoxy-4 phényle.

3. Procédé selon la revendication 1 ou 2, caractérisé en ce qu'on fait réagir des composés III dans lesquels $R^2$ et $R^3$ sont des restes phényle dont les substituants se trouvent en positions 2 et 6, ou des restes naphtyle dont le substituant se situe en position 2.

4. Procédé selon la revendication 1 ou 2, caractérisé en ce qu'on fait réagir des composés III dans lesquels $R^2$ et $R^3$ sont phényle, naphtyle, dichloro-2,6 phényle, diméthoxy-2,6 phényle, méthyl-2 naphtyle, méthoxy-2 naphtyle, diméthyl-2,6 phényle ou triméthyl-2,4,6 phényle.

5. Procédé selon une des revendications 1 à 4, caractérisé en ce qu'on fait réagir des composés III dans lesquels $R^2$ et $R^3$ présentent la même signification.

6. Procédé selon la revendication 1, caractérisé en ce qu'on prépare l'oxyde de phényl bis-(dichloro-2,6 benzoyl) phosphine.

7. Procédé selon la revendication 1, caractérisé en ce qu'on prépare l'oxyde de diméthyl-2,5 phényl bis-(dichloro-2,6 benzoyl) phosphine.

8. Procédé selon la revendication 1, caractérisé en ce qu'on prépare l'oxyde de n-propyl-4 phényl bis-(dichloro-2,6 benzoyl) phosphine.

9. Utilisation des composés préparés selon la revendication 1 en tant que photo-initiateurs dans des masses photopolymérisables.

**Claims for the contracting states BE, CH, NE, FR, GB, IT, LI, LU, NL, SE**

1. Bisacylphosphine oxides of the general formula

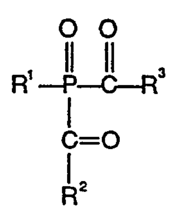

(I)

wherein
$R^1$ stands for a straight-chain or branched $C_{1-18}$ alkyl radical, a cyclohexyl, cyclopentyl, phenyl, naphthyl, or biphenylyl radical,
a cyclopentyl, cyclohexyl, phenyl, naphthyl or biphenylyl radical substituted by F, Cl, Br, I, $C_{1-12}$ alkyl and/or $C_{1-12}$ alkoxyl,
an S- or N-containing 5-membered or 6-membered heterocyclic ring, or
a radical of the general formula:

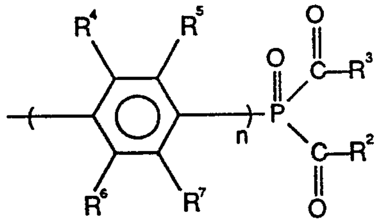

(II)

wherein
n is 1 or 2, and
$R^4$, $R^5$, $R^6$ and $R^7$ are H, $C_{1-4}$ alkyl, $C_{1-4}$ alkoxyl, F, Cl or Br;
$R^2$ and $R^3$, which are the same or different, stand for
a cyclohexyl, cyclopentyl, phenyl, naphthyl, or biphenylyl radical,
a cyclopentyl, cyclohexyl, phenyl, naphthyl or biphenylyl radical substituted by F, Cl, Br, I, $C_{1-4}$ alkyl and/or $C_{1-4}$ alkoxyl, or
an S- or N-containing 5-membered or 6-membered heterocyclic ring; or
$R^2$ and $R^3$ are joined to form a ring containing 4 to 10 carbon atoms and being optionally substituted by 1 to 6 $C_{1-4}$ alkyl radicals.

2. Compounds according to claim 1, characterized in that $R^1$ stands for decyl, phenyl, naphthyl, 4-biphenylyl, 2-methylphenyl, 1-methylnaphthyl, 2,5-dimethylphenyl, 4-propylphenyl, 4-octylphenyl, 4-chlorophenyl or 4-ethoxyphenyl.

3. Compounds according to claim 1 or 2, characterized in that $R^2$ and $R^3$ stand for phenyl radicals whose substituents are in the 2- and 6-positions, or naphthyl radicals whose substituent is in the 2-position.

4. Compounds according to claim 1 or 2, characterized in that $R^2$ and $R^3$ stand for phenyl, naphthyl, 2,6-dichlorophenyl, 2,6-dimethoxyphenyl, 2-methylnaphthyl, 2-methoxynaphthyl, 2,6-dimethylphenyl, or 2,4,6-trimethylphenyl.

5. Compounds according to one of claims 1 to 4, characterized in that $R^2$ and $R^3$ have the same meaning.

6. Bis-(2,6-dichlorobenzoyl)phenylphosphine oxide.

7. Bis-(2,6-dichlorobenzoyl)-2,5-dimethylphenylphosphine oxide.

8. Bis-(2,6-dichlorobenzoyl)-4-n-propylphenylphosphine oxide.

9. A process for preparing the compounds according to claim 1, characterized in that a bisacylphosphine of the general formula

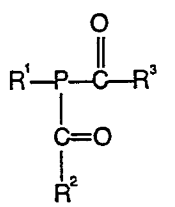

(III)

is oxidized.

10. The use of the compounds of claim 1 as photoinitiators in photopolymerizable compositions.

**Claims for contracting state AT**

1. A process for preparing bisacylphosphine oxides of the general formula

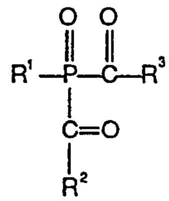

(I)

wherein
$R^1$ stands for a straight-chain or branched $C_{1-18}$ alkyl radical, a cyclohexyl, cyclopentyl, phenyl, naphthyl, or biphenylyl radical,
a cyclopentyl, cyclohexyl, phenyl, naphthyl or biphenylyl radical substituted by F, Cl, Br, I, $C_{1-12}$ alkyl and/or $C_{1-12}$ alkoxyl,
an S- or N-containing 5-membered or 6-membered heterocyclic ring, or
a radical of the general formula:

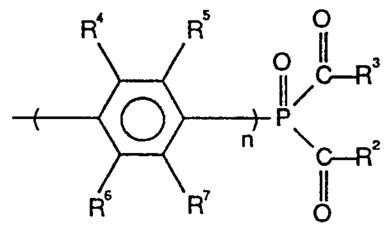

(II)

wherein
n is 1 or 2, and
$R^4$, $R^5$, $R^6$ and $R^7$ are H, $C_{1-4}$ alkyl, $C_{1-4}$ alkoxyl, F, Cl or Br;
$R^2$ and $R^3$, which are the same or different, stand for a cyclohexyl, cyclopentyl, phenyl, naphthyl, or biphenylyl radical,
a cyclopentyl, cyclohexyl, phenyl, naphthyl or bipenylyl radical substituted by F, Cl, Br, I, $C_{1-4}$ alkyl and/or $C_{1-4}$ alkoxyl, or
an S- or N-containing 5-membered or 6-membered heterocyclic ring; or
$R^2$ and $R^3$ are joined to form a ring containing 4 to 10 carbon atoms and being optionally substituted by 1 to 6 $C_{1-4}$ alkyl radicals, characterized in that a bisacylphosphine of the general forumla

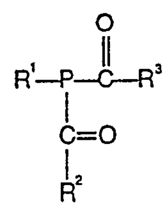

(III)

wherein $R^1$, $R^2$ and $R^3$ have the above-mentioned meanings, is oxidized.

2. A process according to claim 1, characterized in that compounds III are reacted in which $R^1$

stands for decyl, phenyl, naphthyl, 4-biphenylyl, 2-methylphenyl, 1-methyl-naphthyl, 2,5-dimethylphenyl, 4-propylphenyl, 4-octylphenyl, 4-chlorophenyl or 4-ethoxyphenyl.

3. A process according to claim 1 or 2, characterized in that compounds III are reacted in which $R^2$ and $R^3$ stand for phenyl radicals whose substituents are in the 2- and 6-positions, or naphthyl radicals whose substituent is in the 2-position.

4. A process according to claim 1 or 2, characterized in that compounds III are reacted in which $R^2$ and $R^3$ stand for phenyl, naphthyl, 2,6-dichlorophenyl, 2,6-dimethoxyphenyl, 2-methylnaphthyl, 2-methoxynaphthyl, 2,6-dimethylphenyl, or 2,4,6-trimethylphenyl.

5. A process according to one of claims 1 to 4, characterized in that compounds III are reacted in which $R^2$ and $R^3$ have the same meaning.

6. A process according to claim 1, characterized in that Bis-(2,6-dichlorobenzoyl)phenylphosphine oxide is prepared.

7. A process according to claim 1, characterized in that Bis(2,6-dichlorobenzoyl)-2,5-dimethylphenylphosphine oxide is prepared.

8. A process according to claim 1, characterized in that Bis-(2,6-dichlorobenzoyl)-4-n-propylphenylphosphine oxide is prepared.

9. The use of the compounds prepared according to claim 1 as photoinitiators in photopolymerizable compositions.